**Europäisches Patentamt**

**European Patent Office**

**Office européen des brevets**

(19)

(11) Veröffentlichungsnummer : **0 270 661 B1**

## (12) EUROPÄISCHE PATENTSCHRIFT

(45) Veröffentlichungstag der Patentschrift :
**15.01.92 Patentblatt 92/03**

(51) Int. Cl.$^5$ : **A61F 5/01**

(21) Anmeldenummer : **87904487.3**

(22) Anmeldetag : **24.06.87**

(86) Internationale Anmeldenummer :
**PCT/DE87/00289**

(87) Internationale Veröffentlichungsnummer :
**WO 88/00033 14.01.88 Gazette 88/02**

(54) ORTHOPÄDISCHE VORRICHTUNG.

(30) Priorität : **25.06.86 DE 3621510**
**20.06.87 DE 3720767**

(43) Veröffentlichungstag der Anmeldung :
**15.06.88 Patentblatt 88/24**

(45) Bekanntmachung des Hinweises auf die
Patenterteilung :
**15.01.92 Patentblatt 92/03**

(84) Benannte Vertragsstaaten :
**AT BE CH DE FR GB IT LI LU NL SE**

(56) Entgegenhaltungen :
**US-A- 2 439 100**
**US-A- 2 536 454**
**US-A- 2 567 195**
**US-A- 3 055 359**
**US-A- 3 230 952**

(73) Patentinhaber : **F.W. KRAEMER KG**
**Ronsdorfer Strasse 188-190**
**W-5630 Remscheid-Haddenbach (DE)**

(72) Erfinder : **MELCHES, Lothar**
**Bünder Strasse 99**
**W-4986 Rödinghausen (DE)**

(74) Vertreter : **Ostriga, Harald, Dipl.-Ing. et al**
**Patentanwälte Dipl.-Ing. Harald Ostriga**
**Dipl.-Ing. Bernd Sonnet Stresemannstrasse**
**6-8**
**W-5600 Wuppertal 2 (DE)**

EP 0 270 661 B1

EP 0 270 661 B1

## Beschreibung

Die Erfindung betrifft eine orthopädische Vorrichtung für Patienten mit geschwächtem oder ausgefallenem Fußhebermuskel entsprechend dem Oberbegriff des Patentanspruchs 1.

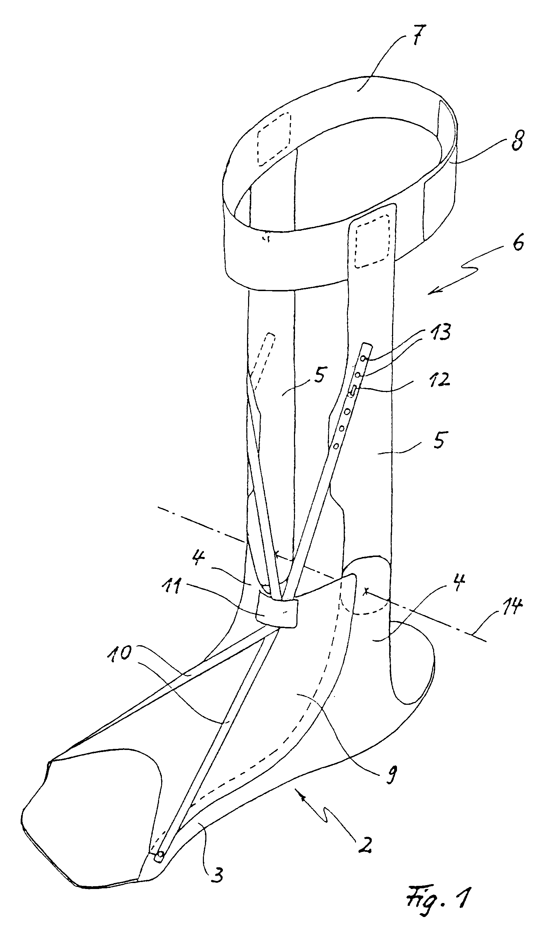

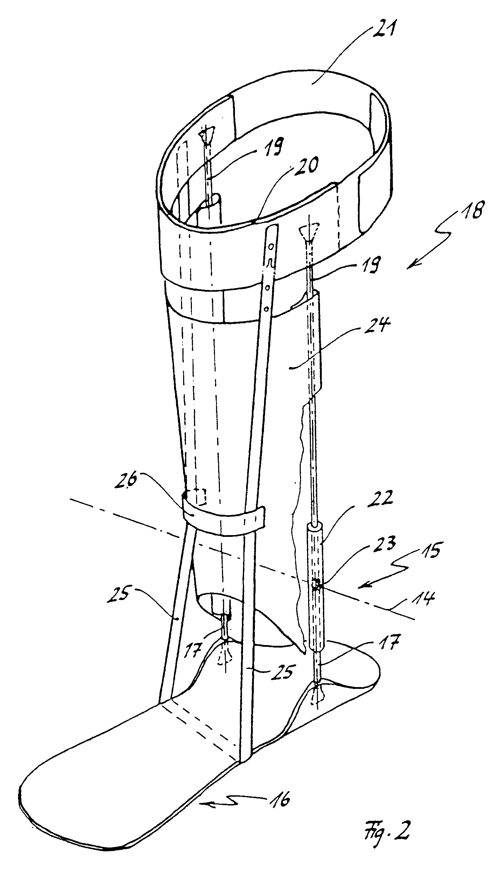

Eine derartige orthopädische Vorrichtung ist durch die US-A- 32 30 952 bekannt, die einen Hüftgelenk- und Beckenspange enthaltenden Komplett-Lähmungsapparat für beide Beine beschreibt. Die US-A 32 30 952 (s. dort Fig. 1 und 2) befaßt sich mit der besonderen Gelenksituation eines solchen Lähmungsapparates, was ebenso für die Fig. 3-6 gilt, während Fig. 9 im Zusammenhang mit den Fig. 7 und 8 den Lähmungsapparat mehr schematisch insgesamt darstellen. Dort ist auch eine für derartige Lähmungsapparate erforderliche Fersenfeder gezeigt, die in den auf die Gelenksituation beschränkten Fig. 1-2 weggelassen ist. Auch wird im Zusammenhang mit den Fig. 1 und 9 der US-A 32 30 952 deutlich, daß die dort gezeigten Halblaschen 15 bzw. 15' nach hinten geschlossen sind. Allein schon aus diesem Grunde ist der bekannte Lähmungsapparat nur von vorn einsteigbar .

Unter Berücksichtigung der orthopädischen Vorrichtung gemäß der US-A 32 30 952, liegt der Erfindung die Aufgabe zugrunde, eine in ihrer Funktion und Tragweise verbesserte Fußheberschiene, auch als "Peronaeusschiene" bezeichnet, für Patienten mit geschwächtem oder ausgefallenem Fußhebermuskel zu schaffen.

Bekannte Fußheberschienen sind mit schwerwiegenden Nachteilen behaftet. So besteht beispielsweise eine offenkundig vorbekannte Fußheberschiene aus einer Fußschale in Art einer Einlagensohle, an deren hinterem Fersenende, etwa vergleichbar der US-A 32 30 952 (vgl. dort Fig. 9 Position 51) eine langgestreckte Blattfeder aus Federstahl oder federndem Kunststoff befestigt ist, die sich auf der Rückseite des Beines als Beinschiene bis in Höhe der Wade erstreckt und mit ihrem oberen Ende mittels einer von hinten das Bein umgreifenden Wadenschelle an der Wade des Patienten anliegt.

Jene Blattfeder soll beim Gehen gewährleisten, daß der Fuß des Patienten beim Abheben und/oder beim Aufsetzen auf den Boden im wesentlichen einen rechten Winkel zum Bein einimmt, so daß das Schleifen der Fußspitze auf dem Boden - und damit ein Stolpern - vermieden wird. Gleichzeitig soll aber bei der Gewichtsbelastung des Fußes unmittelbar nach dem Aufsetzvorgang die Blattfeder nachgeben, so daß sich der Winkel zwischen Fuß und Bein öffnet und die Fußsohle vollflächig auf den Boden auflastet.

Aus diesem Bewegungsablauf resultiert bei den bekannten Vorrichtungen der Nachteil, daß die Wadenschelle am Bein eine Scheuerbewegung ausführt, da sich die Position der Wadenschelle beim Öffnen des Winkels zwischen Fuß und Bein verändert. Auch ist es schmerzhaft, wenn sich die Wadenschelle bzw. das obere Ende der Blattfeder in den Wadenmuskel eindrückt. Um dieses zu verhindern, muß die Blattfeder mit einer übergroßen Länge ausgestattet sein, die gewährleisten soll, daß die Wadenschelle oberhalb des Wadenmuskels positioniert ist. Für den Patienten ist das weniger angenehm, da er z.B. in der heißen Sommerzeit auf eine lange Beinbekleidung angewiesen ist, welche die orthopädische Hilfsvorrichtung überdeckt.

Weiterhin ist nachteilig, daß die hinten an der Ferse bzw. an dem Bein angeordnete Blattfeder der bekannten Vorrichtung beim Bewegungsablauf, insbesondere beim Öffnen des Winkels zwischen Fuß und Bein, eine erhöhte Schubkraft auf den Fuß ausübt, die diesen innerhalb des Schuhs nach vorne drückt und nur durch ein festes Schuhwerk aufgefangen werden kann. Auch verhindert die an der Ferse verlaufende Blattfeder einen sicheren Kontakt zwischen der Ferse und dem Schuhwerk bzw. der hinteren Schuhkappe, so daß viele Patienten bei den bekannten Vorrichtungen einen mangelnden festen Sitz der Ferse im Schuh beklagen.

Im Bewußtsein der Nachteile des Bekannten wird, ausgehend von dem Lähmungsapparat gemäß der US-A 32 30 952, die vorzitierte Aufgabe erfindungsgemäß dadurch gelöst, daß die orthopädische Vorrichtung als Fußheberschiene ausgebildet ist, deren beide seitliche Außenstücke oben endseitig mittels einer geschlossenen Wadenschelle miteinander verbunden sind, welche nach hinten zu öffnen ist, so daß die Beinschiene mit Fußschale, die nach hinten offen ausgeführt sind, von hinten einsteigbar ist und daß zwei auf der Vorderseite der Fußheberschiene angeordnete Gummizugbänder mittels eines Querbandes geführt sind, welches etwa in Höhe der Schwenkachse der Schwenkgelenke zwischen den Außenstücken der Beinschiene bzw. den Ansatzstücken angeordnet ist.

Dadurch, daß zwei auf der Vorderseite der Fußheberschiene angeordnete Gummizugbänder mittels eines Querbandes geführt sind, welches etwa in Höhe der Schwenkachse der Schwenkgelenke zwischen den Außenstücken der Beinschiene und/oder den Ansatzstücken angeordnet ist, vermeidet die Erfindung eine nachteilige Schubkraft auf den Fuß beim Öffnen des Winkels zwischen Fuß und Bein und schafft somit die Voraussetzung für die wesentliche Ausbildung der Erfindung, bei der die Beinschiene und die Fußschale in vorteilhafter Weise nach hinten offen ausgeführt sind.

Hierdurch wird nicht nur der Vorteil erreicht, daß der Patient von hinten in die orthopädische Vorrichtung einsteigen kann, sondern es wird auch der natürliche direkte Kontakt zwischen Ferse und Schuhwerk sichergestellt, so daß der Fuß des Patienten in dem Schuh trotz Verwendung einer orthopädischen Hilfsvorrichtung nicht schlupfen kann.

2

In weiterer Ausgestaltung der Erfindung ist die Zugkraft der Gummizugbänder durch wählbare Längen der Gummizugbänder zwischen ihren Befestigungen an der Beinschiene einerseits und an der Fußschale andererseits einstellbar.

Die konstruktive Ausbildung der Schwenkgelenke kann in jeder bekannten Weise erfolgen, jedoch wird eine zusätzliche erfinderische Maßnahme darin gesehen, ein Schwenkgelenk auszubilden, das aus einer elastischen Hülse mit zwei von den gegenüberliegenden Enden in die Hülse eingeschobenen Stangen besteht, die zum einen an den Außenstücken der Beinschiene und zum anderen an den Ansatzstücken der Fußschale befestigt sind.

Dabei sieht eine zweckmäßige Ausführungsform der Erfindung vor, daß die Außenstücke der Beinschiene und die Ansatzstücke der Fußschale durch die entsprechend verlängerten Stangen des Schwenkgelenkes selbst gebildet sind.

Eine solche erfindungsgemäße Vorrichtung ist besonders leicht zu tragen und sie hat den wesentlichen Vorteil, daß das neue Schwenkgelenk nicht nur eine Schwenkbewegung nach allen Richtungen zuläßt, sondern auch in axialer Richtung der Stangen auseinandergleiten und wieder zusammengeschoben werden kann, wie dies beim Gehen in schwierigem Gelände wünschenswert ist.

Grundsätzlich gilt, daß der Patient trotz der Verwendung der orthopädischen Hilfsvorrichtung so gehen sollte, daß er stets einen vollflächigen Bodenauftritt hat. Ist der Boden jedoch uneben und bildet z.B. eine zur Seite geneigte schiefe Ebene, dann ist der vollflächige Bodenauftritt bei den bisher üblichen Schwenkgelenken nicht mehr gewährleistet. Das neugeschaffene Hülsen-Stangen-Schwenkgelenk vermag aber auch diesem Bewegungsablauf uneingeschränkt zu folgen.

Die elastische Hülse des neuen Schwenkgelenkes kann aus einem dauerelastischen Kunststoff oder aber auch aus einer elastischen Spiralfeder, die in Hülsenform gewickelt ist, bestehen. Die Stangen des Schwenkgelenkes sind zweckmäßigerweise aus Metall und bilden zugleich die Außenstücke der Beinschiene und die Ansatzstücke der Fußschale.

Dies hat auch den Vorteil, daß der Orthopädiemechaniker problemlos durch Abschneiden bzw. Ablängen der Stangen das Schwenkgelenk der orthopädischen Vorrichtung exakt an das anatomische Fußgelenk des Patienten anpassen kann.

Besonders zweckmäßig ist es, in der Hülse des neuen Schwenkgelenkes zwischen den in die Hülse eingeschobenen Stangenenden ein Gleitlager, z.B. in Form einer Kunststoffkugel, anzuordnen.

Nachfolgend werden zwei Ausführungsbeispiele der Erfindung anhand der Zeichungen näher beschrieben. Es zeigen:

Fig. 1 in perspektivischer Darstellung eineerste Ausführungsform der Erfindung mit den bis dato üblichen Schwenkgelenken,

Fig. 2 eine zweite Ausführungsform der Erfindung mit einem neuen erfindungsgemäßen Schwenkgelenk.

Das in Fig. 1 dargestellte erste Ausführungsbeispiel zeigt die Fußschale 2 mit seitlich hochgezogenen Fußbetträndern 3, die im Bereich des Fußgelenkes in angeformte seitliche Ansatzstücke 4 übergehen.

An den seitlichen Ansatzstücken sind jeweils seitliche Außenstücke 5 der Beinschienen 6 angelenkt, wobei die oberen Enden der Außenstücke 5 mit einem textilen Schellenband 7, das mit einem Klettverschluß 8 versehen ist, relativ zueinander festgelegt und am Bein des Patienten gehalten sind.

Bei geöffnetem Klettverschluß 8 kann der Patient problemlos von hinten in die orhopädische Vorrichtung einsteigen bis der Spann seines Fußes an einem textilen, atmungsaktiven Stretchgewebe 9 zur Anlage kommt, das bei dem dargestellten Ausführungsbeispiel an den Fußbetträndern 3 und dem vorderen Rand des Ansatzstückes 4 befestigt ist und den Fuß überspannt. Ein solches Stretchgewebe muß nicht den dargestellten Zuschnitt haben und kann z.B. auch zusätzlich auf der Vorderseite der Beinschiene 6 zwischen den beiden Außenstücken 5 vorgesehen sein und das Schienbein des Patienten als Stützband überspannen.

Die Gummizugbänder 10 sind auf der Vorderseite der Vorrichtung angeordnet und mittels eines Querbandes 11 geführt, das in etwa in Höhe der Schwenkachse 14 der Schwenkgelenke angeordnet ist, so daß die Gummizugbänder eng am Bein des Patienten verlaufen und dennoch beim Gehen, d. h. beim Verändern des Öffnungswinkels zwischen Fußschale 2 und Beinschiene 6 nicht stören.

Die Befestigung der Gummizugbänder 10 erfolgt bei dem dargestellten Ausführungsbeispiel an den Außenstücken 5 der Beinschiene 6, und zwar in einfachster Weise mittels eines kleinen Hakens 12, auf den das jeweilige Gummizugband mit einem seiner vorgestanzten Löcher 13 aufgehakt wird. Je nach Wahl des Loches 13 kann die Zugkraft des Gummizugbandes der individuellen Funktion und dem Wohlbefinden des Patienten angepaßt werden.

Die seitlichen Außenstücke 5 der Beinschiene 6 sind mit den vertikal sich erstreckenden Ansatzstücken 4 der Fußschale 2 jeweils durch ein Schwenkgelenk verbunden, deren jeweilige Schwenkachsen durch die eingezeichnete Achse 14 verdeutlicht sind. Die Achse 14 soll möglichst koaxial mit dem anatomischen Fußgelenk des Patienten angeordnet sein. Dementsprechend wird die Achse 14 erst bei der individuellen Anpassung der erfindungsgemäßen Vorrichtung an den Fuß des Patienten durch den Orthopädiemechaniker genau positioniert, was in einfachster Weise z.B. durch Einsetzen einer Niete erfolgen kann, die die in Fig. 1 dargestellten Außenstücke 5 der Beinschiene mit den Ausatzstücken 4 der Fußschale verbindet.

Fig. 2 zeigt ein zweites Ausführungsbeispiel der Erfindung mit dem erfindungsgemäßen neuen Schwenkgelenk 15. Auch hier wieder ist die Schwenkachse des Schwenkgelenkes mit 14 bezeichnet.

Bei diesem Ausführungsbeispiel besitzt die Fußschale 16 zwei vertikal sich erstreckende Ansatzstücke in Form der Stangen 17, die mit der Fußschale 16 starr verbunden sind und zweckmäßigerweise, da die Fußschale meistens aus Kunststoff gefertigt ist, in die hochgezogenen Ränder der Fußschale mit eingeformt sind.

Die beiden seitlichen Außenstücke der Beinschiene 18 bestehen bei diesem Ausführungsbeispiel ebenfalls aus zwei Stangen 19, deren obere Enden mittels einer das Bein umgreifenden Brücke 20, z.B. aus Kunststoff, relativ zueinander gehalten sind. Diese Brücke wird dann wieder mit einem Schellenband 21 am Bein des Patienten befestigt.

Das erfindungsgemäße neue Schwenkgelenk 15 besteht aus der elastischen Hülse 22, in die von den gegenüberliegenden Enden jeweils die Stangen 19 und 17 eingeschoben worden sind. In der Hülse ist zwischen den eingeschobenen Stangenenden ein Gleitlager, z.B. in Form der Kunststoffkugel 23, angeordnet, das genau in der Schwenkachse 14 positioniert ist.

In die in Fig. 2 dargestellte Vorrichtung kann der Patient problemlos von hinten einsteigen, da die Vorrichtung nach hinten offen ist und lediglich auf der Vorderseite eine Anlagefläche 24 vorhanden ist, die z.B. aus einem textilen Stretchgewebe oder aus hautsympathischem Leder gefertigt sein kann und an den Stangen 19 und 17 der Beinschiene bzw. der Fußschale befestigt ist.

Auch bei diesem Ausführungsbeispiel sind auf der Vorderseite der Vorrichtung Gummibänder 25 vorgesehen, die mittels eines Querbandes 26 an der vorderen Anlagefläche 24 geführt sind.

## Patentansprüche

1. Orthopädische Vorrichtung für Patienten mit geschwächtem oder ausgefallenem Fußhebermuskel,
   – mit einer Fußschale (2, 16) in Art einer Einlegesohle, welche beidseitig jeweils ein sich vertikal erstreckendes Ansatzstück (4, 17) besitzt, das mindestens bis zum anatomischen Fußgelenk hochgezogen ist,
   – mit einer Beinschiene, welche zwei seitliche Außenstücke (5, 19) aufweist, die jeweils mittels eines Schwenkgelenkes (14, 15) an einem der Ansatzstücke (4, 17) der Fußschale (2, 16) koaxial mit dem anatomischen Fußgelenk angelenkt sind,
   – und daß mindestens eine Zugfeder (10, 25) auf der Vorderseite der Vorrichtung angeordnet ist, die mit ihrem einen Ende an der Beinschiene (6, 18) und mit ihrem anderen Ende an der Fußschale (2, 16) befestigt ist,
   dadurch gekennzeichnet, daß die orthopädische Vorrichtung als Fußheberschiene (6, 18) ausgebildet ist, deren beide seitliche Außenstücke (5, 19) oben endseitig mittels einer geschlossenen Wadenschelle (7, 21) miteinander verbunden sind, welche nach hinten zu öffnen ist, so daß die Beinschiene (6, 18) mit Fußschale (2, 16), die nach hinten offen ausgeführt sind, von hinten einsteigbar ist und daß zwei auf der Vorderseite der Fußheberschiene (6, 18) angeordnete Gummizugbänder (10, 25) mittels eines Querbandes (11, 26) geführt sind, welches etwa in Höhe der Schwenkachse der Schwenkgelenke (14, 15) zwischen den Außenstücken (5, 19) der Beinschiene (6, 18) bzw. den Ansatzstücken (4, 17) angeordnet ist.

2. Vorrichtung nach Anspruch 1, dadurch gekennzeichnet, daß die Zugkraft der Gummizugbänder (10, 25) durch wählbare Längen der Gummizugbänder zwischen ihren Befestigungen an der Beinschiene (6, 18) einerseits und an der Fußschale andererseits (2, 16) einstellbar ist.

3. Vorrichtung nach Anspruch 1 oder Anspruch 2, gekennzeichnet durch ein Schwenkgelenk (15), das aus einer elastischen Hülse (22) mit zwei von den gegenüberliegenden Enden in die Hülse eingeschobenen Stangen (19, 17) besteht, die zum einen an den Außenstücken der Beinschiene (18) und zum anderen an den Ansatzstücken der Fußschale (16) befestigt sind.

4. Vorrichtung nach Anspruch 3, dadurch gekennzeichnet, daß die Außenstücke der Beinschiene und die Ansatzstücke der Fußschale durch die Stangen (19 bzw. 17) des Schwenkgelenkes (15) gebildet sind.

5. Vorrichtung nach Anspruch 3 oder 4, dadurch gekennzeichnet, daß in der Hülse (22) zwischen den in die Hülse eingeschobenen Stangenenden (19, 17) ein Gleitlager (23) angeordnet ist.

EP 0 270 661 B1

## Claims

1. Orthopaedic device for patients with an impaired or non-functioning flexor muscle of the foot,
   – having a foot shell (2, 16) in the manner of an insole, which has on either side a vertically extending attachment piece (4, 17) raised up at least as far as the anatomical ankle joint,
   – having a leg splint with two lateral outer pieces (5, 19), which are each hinged coaxially to the anatomical ankle joint by means of a swivel joint (14, 15) on one of the attachment pieces (4, 17) of the foot shell (2, 16),
   – and in that at least one tension spring (10, 25) is disposed on the front of the device and is fastened by one end to the leg splint (6, 18) and by its other end to the foot shell (2, 16),
   characterised in that the orthopaedic device takes the form of a foot flexing splint (6, 18), the two lateral outer pieces (5, 19) of which are connected to one another at their top end by means of a closed calf clip (7, 21) which is to be rear-opening so that the leg splint (6, 18) with foot shell (2, 16), which are designed to be open towards the rear, may be entered from the rear, and in that two rubber tension bands (10, 25) arranged on the front of the foot flexing splint (6, 18) are guided by means of a transverse band (11, 26) which is disposed approximately at the level of the swivelling axis of the swivel joints (14, 15) between the outer pieces (5, 19) of the leg splint (6, 18) and/or the attachment pieces (4, 17).

2. Device according to claim 1, characterised in that the tensile force of the rubber tension bands (10, 25) is adjustable by means of selectable lengths of the rubber tension bands between their fastenings on the leg splint (6, 18) on the one hand and on the foot shell (2, 16) on the other hand.

3. Device according to claim 1 or claim 2, characterised by a swivel joint (15) comprising an elastic sleeve (22) with two rods (19, 17), which are inserted into opposite ends of the sleeve and are fastened on the one hand to the outer pieces of the leg splint (18) and on the other hand to the attachment pieces of the foot shell (16).

4. Device according to claim 3, characterised in that the outer pieces of the leg splint and the attachment pieces of the foot shell are formed by the rods (19 and 17) of the swivel joint (15).

5. Device according to claim 3 or 4, characterised in that a sliding bearing (23) is disposed in the sleeve (22) between the rod ends (19, 17) inserted into the sleeve.

## Revendications

1. Dispositif orthopédique pour patient dont le muscle releveur du pied est hypotonique ou défaillant,
   – comprenant un plateau de pied (2, 16) présentant la forme d'une semelle intérieure, qui possède sur chaque côté une patte (4, 17) s'étendant verticalement, qui est prolongée vers le haut au moins jusqu'à l'articulation anatomique du pied,
   – comprenant un tuteur de jambe qui présente deux pièces extérieures latérales (5, 19) dont chacune est articulée au moyen d'une articulation (14, 15) à l'une des pattes (4, 17) du plateau de pied (2, 16), coaxialement à l'articulation anatomique du pied,
   – et comprenant au moins un ressort de traction (10, 25) disposé sur le côté avant du dispositif, qui est fixé au tuteur de jambe (6, 18) par l'une de ses extrémités et au plateau de pied (2, 16) par son autre extrémité,
   caractérisé en ce que le dispositif orthopédique est constitué sous la forme d'une orthèse antisteppage (6, 18) dont les deux pièces extérieures latérales (5, 19) sont reliées l'une à l'autre en haut, à leur extrémité, au moyen d'un bandage de mollet fermé (7, 21) qui peut s'ouvrir vers l'arrière, de sorte que le tuteur de jambe (6, 18), avec le plateau de pied (2, 16), d'une constitution ouverte vers l'arrière, peut être chaussé par l'arrière et en ce que deux tendeurs en caoutchouc (10, 25) disposés sur le côté avant de l'orthèse antisteppage (6, 18) sont guidés au moyen d'une bande transversale (11, 26) qui est disposée à peu près à la hauteur de l'axe de rotation des articulations (14, 15) entre les pièces extérieures (5, 19) du tuteur de jambe (6, 18) et les pattes (4, 17).

2. Dispositif selon la revendication 1, caractérisé en ce que la force de traction des tendeurs en caoutchouc (10, 25) peut être réglée grâce à la possibilité de régler les longueurs des tendeurs en caoutchouc entre leurs fixations sur le tuteur de jambe (6, 18), d'une part et le plateau de pied (2, 16) d'autre part.

3. Dispositif selon la revendication 1 ou la revendication 2, caractérisé par une articulation (15) qui est composée d'un manchon élastique (22) renfermant deux tringles (19, 17) enfilées dans le manchon par les extrémités opposées, et qui sont fixées, d'une part, aux pièces extérieures du tuteur de jambe (18) et, d'autre part, aux pattes du plateau de pied (16).

4. Dispositif selon la revendication 3, caractérisé en ce que les pièces extérieures du tuteur de jambe et les pattes du plateau de pied sont formées par les tringles (19 et 17 respectivement) de l'articulation (15).

5

5. Dispositif selon la revendication 3 ou 4, caractérisé en ce qu'un palier lisse (23) est disposé dans le manchon (22) entre les extrémités des tringles (19, 17) enfilées dans le manchon.

Fig. 1

Fig. 2